# EUROPEAN PATENT SPECIFICATION

(11) **EP 2 842 568 B1**
(45) Date of publication and mention of the grant of the patent: **14.11.2018**
(21) Application number: 13785267.9
(22) Date of filing: 12.04.2013
(51) Int. Cl.: A61K 38/28, A61K 38/16, A61K 31/573, A61K 39/395, A61K 31/52, A61K 38/13, A61P 3/10, A61P 37/02

(54) **PHARMACEUTICAL COMPOSITION FOR TREATING AND/OR PREVENTING TYPE I DIABETES AND APPLICATION THEREOF**
PHARMAZEUTISCHE ZUSAMMENSETZUNG ZUR BEHANDLUNG UND/ODER PRÄVENTION VON TYP-I-DIABETES UND ANWENDUNG DAVON
COMPOSITION PHARMACEUTIQUE POUR TRAITER ET/OU PRÉVENIR LE DIABÈTE TYPE I ET SON APPLICATION

(30) Priority: 13.04.2012 CN 201210108159
(43) Date of publication of application: 04.03.2015
(73) Proprietor: Beijing Advaccine Biotechnology Co., Ltd., Haidian, Beijing 100085 (CN)
(72) Inventor: WANG, Bin, Beijing 100085 (CN); ZHENG, Guoxing, Beijing 100085 (CN); GENG, Shuang, Beijing 100085 (CN); WANG, Yizhong, Beijing 100085 (CN); YU, Qingling, Beijing 100085 (CN)
(74) Representative: Graf von Stosch, Andreas
(86) International application number: PCT/CN2013/000429
(87) International publication number: WO 2013/163887

(56) References cited:
- WO-A1-2006/081669
- WO-A2-2005/076965
- US-A1- 2003 211 113
- Y. KANG ET AL: "Cutting Edge: Immunosuppressant as Adjuvant for Tolerogenic Immunization", THE JOURNAL OF IMMUNOLOGY, vol. 180, no. 8, 15 April 2008 (2008-04-15), pages 5172-5176, XP055250658, US ISSN: 0022-1767, DOI: 10.4049/jimmunol.180.8.5172
- D FRANCHIMONT: "Overview of the Actions of Glucocorticoids on the Immune Response: A Good Model to Characterize New Pathways of Immunosuppression for New Treatment Strategies", ANNALS OF THE NEW YORK ACADEMY OF SCIENCES, vol. 1024, no. 1, 1 June 2004 (2004-06-01) , pages 124-137, XP055250657, US ISSN: 0077-8923, DOI: 10.1196/annals.1321.009
- DELONG T ET AL: "Islet Amyloid Polypeptide is the Antigen Target for the Highly Diabetogenic CD4+ T Cell Clone BDC-5.2.9", CLINICAL IMMUNOLOGY, ACADEMIC PRESS, US, vol. 135, 1 January 2010 (2010-01-01), page S19, XP027048861, ISSN: 1521-6616 [retrieved on 2010-01-01]
- MATTHEWS, J.B. ET AL.: 'Developing combination immunotherapies for type 1 diabetes: recommendations from the ITN-JDRF type I diabetes combination therapy assessment group' CLINICAL AND EXPERIMENTAL IMMUNOLOGY vol. 160, no. 2, May 2010, pages 176 - 184, XP055172497
- BRESSON, D. ET AL.: 'Genetic-induced variations in the GAD65 T-cell repertoire governs efficacy of anti-CD3/GAD65 combination therapy in new-onset type 1 diabetes' MOLECULAR THERAPY vol. 18, no. 2, February 2010, pages 307 - 316, XP002667930
- BRESSON, D. ET AL.: 'Anti-CD3 and nasal proinsulin combination therapy enhances remission from recent-onset autoimmune diabetes by inducing Tregs' THE JOURNAL OF CLINICAL INVESTIGATION vol. 116, no. 5, May 2006, pages 1371 - 1381, XP008167683
- ZHANG, XIN: 'Research Progress on Islet Amyloid Polypeptide' CHINESE BULLETIN OF LIFE SCIENCE vol. 22, no. 6, June 2010, pages 567 - 574, XP008175282

## Description

### Scope

The present invention relates to vaccines compositions and their use for therapeutic and/or prevention of Type I diabetes.

### Background

Expert of World health Organization (WHO) predicted that diabetes would be the major threat to health in 21 century , even more dangerous threat than avian influenza and AIDS especially in Asia. WHO estimated that the diabetes patient will increase to around 200 million in 2010 and the number will be more than 330 million in 2025. Under the analysis of current situation, in next 10 years, 60% of patients will appear in Asia. As reported, world widely, most diabetes patients are in West Pacific area including China and East South of Asia which also including India. For the 5 countries, which have tremendous number of diabetes patient, there are 4 countries located in Asia.

During the development to wealth in China, the new incidence of diabetes increased years by years under the influence of nutrient like lipid, sugar taking and increasing number of ageing. In most area of China, the average increased incidence reached to 1/1000 compared with 90's of 20 century. The expert from China estimated that this increasing would be worse than what WHO anticipated In the future 10 years. According to the investigation in China, there are more than 25 million diabetes patients and 35 million patients with abnormal glucose tolerance. That means more than 60 million people with age over 20 will be affect by diabetes, in which the incidence of diabetes in rich areas and cities is higher than poorer areas and countryside, the overweight higher than the people with regular weight and the elders higher than young people. Also, new diabetes case appeared among people at age around 40, in which, the incident in those patients with age of more than 40 is about 87% of total diabetes patients and the peak age is arround 50-70. Regarding the current age tendency, there exists variety markets with tremendous potential for diabetes drug developments.

Type I diabetes is a kind of autoimmune disease due to the malfunction of islet, which is characterized by invading of CD4⁺, CD8⁺T cells and macrophages into islet thus the insulin producing beta cells were destroyed by those invading. About 5-10% of diabetes account for TID (ADA [American Diabetes Association].1997. Report of the expert committee on the diagnosis and classification of diabetes mellitus. Diabetes Care 20:1183-1197;Atkinson MA, Leiter EH. 1999. The NOD mouse model of type I diabetes: As good as it gets? Nature 5:60601-604). The main mechanism of the TID is characterized by destroyed insulin producing cells by the auto-reactive T cell, which characterized by the CD4⁺, CD8⁺T cells and macrophages invading into the islet (Atkinson MA, Maclaren NK. 1994. The pathogenesis of insulin-dependent diabetes mellitus. N Engl J Med 331:1428-1436; Benoist C, Mathis D. 1997. Autoimmune diabetes: Retrovirus as trigger, precipitator or marker? Nature 388:833-834; Bjork S. 2001.The cost of diabetes and diabetes care. Diabetes Res Clin Pract 54(Suppi 1):13-18).

The inflammation was found in islet tissue of TID patient. After the lymphocyte invaded into islet of TID patient, the ICA, auto-reactive T cell against insulin, carboxy-peptidase and HSP were found in those patients.

Kang et al. (Journal Immunol. 2008, 180: 5172-5176) teaches immunosuppressants as adjuvants for tolerogenic immunization. Dexamethasone-augmented immunization induced long-term persistent, Agspecific regulatory T cells responsive to recall Ags. Kang et al. discloses administration of an insulin peptide and dexamethasone in a weight ratio of 1:8.

WO 2006/081669 teaches methods for the treatment of new onset Type I diabetes in mammals. The method comprises administering (a) anti-T cell therapy to the mammal and administering (b) an autoantigen, wherein (a) and (b) are administered concurrently or sequentially. More specifically, WO 2006/081669 teaches administration of an anti-CD3 antibody and GAD65 as an auto-antigen.

The interaction of insulin B chain (aa 9-23) with MHC-II that named as I-Ag7 was experimentally demonstrated. The TID or insulin dependent diabetes is a kind T cell mediated disease and as the result of destroyed islet with high glucose in blood due to self reactive cells. The B chain of insulin may be the auto-antigen candidate responsible for disease (Devendra, D. et al. Diabetes 54, 2549, 2005; Starwalt, S. et al. Protein Eng. 16, 147, 2003; Lee, L. et al. PNAS 102, 15995, 2005;). The further experiment demonstrated that peptide 15-23 of insulin B chain could be recognized by T cells, and can be detected for production of interferon β and IL-17 in CD4 or CD8 T cell among TID patients. Other evidence shown that this peptide could react with CTL specific against to insulin B chain but not CD8 T cells in spleen (Hu, C. et al. J. Clin. Invest. 117, 3857, 2007; Amrani, A. et al. Nature 406, 739, 2000). Experiment demonstrated that the insulin C chain is another autoimmune antigen (Arif, T. I. Tree, T. P. Astill et al. Autoreactive T cell responses show proinflammatory polarization in diabetes but a regulatory phenotype in health. The Journal of Clinical Investigation, vol. 113, no. 3, pp. 451-463, 2004).

Till to 1990, Beakkeskov demonstrated the 64 K antibody existed in TID patients' serum is autoreactive antibody and T cells, thus the GAD is considered as the key antigen responsible for TID (Immune modulation for prevention of type I diabetes mellitus. Itamar Raz, Roy Eldor and Yaakov Naparstek. TRENDS in Biotechnology 23:128, 2005; Enee, E. et al. J Immunol 180, 5430, 2008; Xiurong Long, Wenbing Du, Zhongpu Su, Qinzheng Wei. Detection of glutamic acid decarboxylase antibodies in children with diabetes mellitus. Chinese Journal of Pediatrics. 1998, Vol 10.).

On the other hand, lots of reports demonstrated that the protein aggregations induced by islet amyloid polypeptide (IAPP) could destroy the membrane structure of islet beta cell, could induce apoptosis, could cause dysfunctions of islet beta cells and activate immune attacking of islet beta cells. And this protein aggregation was considered as the one of main cause for diabetes. The latest results showed that inhibiting the formation of IAPP could decrease the apoptosis of islet beta cell and increase the possibility of islet transplantation. As the consequence, the IAPP become the other promising target for TID drug development.

Currently, the treatment of TID mainly relies on insulin up-taking in which the patient needs daily injections of insulin. This treatment method is inconvenient for patients and may cause allergy and infections around the injection site. Also, insulin injection method can only partially lower the symptom of high glucose but cannot restore pancreatic function and prevents the pancreatic attack by auto-reactive T cells, thus cannot control the long term neopathy. Considering the complication of diabetes, only 35-90% could be decreased by insulin injection approach. So the diabetes patients suffer those painful inconvenient and side effects. Therefore, it is necessary to develop the new approach to restore or improve the pancreatic function by inhibiting the autoimmune responses towards the islet cells and let to potential cure in those TID patients especially for those young patient to improve their life quality.

Since TID is an autoimmune disease caused by auto-reactive T cells, the immuno-suppressive agents were broadly used in clinic such as Dexamethasone (Dex), tacrolimus (FK506), cyclosporine (CsA), Mycophenolate mofetil (MMF), azathiopurine (Aza), prednisone (Pred), Methylprednisolone (MP), etc, and antibodies against anti lymphocyte globulin (ALG) and anti CD4 monoclonal antibody (OKT4). Those treatment approaches are nonspecific and may cause serious side effects to the patients. On the other hand, these treatments are very expensive in which the patients will spend several billion dollars each year. Also, those agents are non-specific immuno-suppressive agents and have toxicity or side effect if large dose were given. On the other hand, it may cause multiple neopathy and malfunction of organs due to the over inhibition of immune response. So it is urgent to develop target-specific drugs without toxicity or with minimum side effects.

The purpose of this invention is to provide vaccine compositions.

This invention provides active compositions as claimed by claim 1, comprising as the active ingredient:
1) Mixture of protein antigen and immune suppressive agent(s), wherein the weight ratio of the protein antigen and the immune suppressive agent(s) is 1:1 to 10:1.
2) Disclosed mixture of TID epitope peptides and immune suppressive agent(s), wherein the weight ratio of the epitope peptides and the immune suppressive agent(s) is 1:1.

Claim 1 defines the protein antigen as insulin or a fragment thereof and the immune suppressive agent to be selected from the group consisting of dexamethasone (Dex), tacrolimus (FK506), cyclosporine (CsA), prednisone (Pred), methylprednisolone (MP), and combinations thereof.

The active component of TID protein antigen, epitope peptides and immune suppressive agents can be packaged separately or packaged as one formulation.

Disclosed immune suppressive agents include but are not limited to Dex, FK506, CsA, MMF, Aza, Pred, MP, anti CD4 monoclonal antibody and anti CD3 monoclonal antibody. At least one of those agents described as the immune suppressive agents for TID. Epitope peptides refer to single epitope peptide and multi epitopes polypeptide for TID.

In which, listed insulin may come from biological isolation of human, dog, cat, and genetic engineering recombinant protein. The insulin from human can be used for treating TID of cat and dog since insulin gene between human, cat, dog and mouse are very similar, in which, the homology of human and mouse is 95%, human and cat is 84%, human and cat is 89%.

The listed epitope peptides can be from human, cat, dog and can be chemically synthesized.

The listed protein antigen for TID can be specific to rh-insulin. The listed epitope peptides from rh-insulin refer to sequence 1 (this peptide named as B9-23), or sequence 2 (this peptide named as B15-23), or sequence 3 (this peptide named as C peptide), or sequence 12 (this peptide named as B23-29), or sequence 13 (this peptide named as B10-C5).

The listed protein antigen for TID can be specific to dog insulin. The listed epitope peptides from dog insulin refer to sequence 4 (this peptide named as B9-23), or sequence 5 (this peptide named as B15-23), or sequence 12 (this peptide named as B23-29), or sequence 13 (this peptide named as B10-C5).

The listed protein antigen for TID can be specific to cat insulin. The listed epitope peptides from cat insulin refer to sequence 6 (this peptide named as B9-23), or sequence 7 (this peptide named as B15-23), or sequence 12 (this peptide named as B23-29), or sequence 13 (this peptide named as B10-C5).

Also disclosed is protein antigen for TID specific to human GAD65. The listed epitope peptide from human GAD65 refer to sequence 8 (this peptide named as G114-123).

The disclosed protein antigen for TID can be specific to human IAPP. The disclosedepitope peptide from human IAPP refer to sequence 9 (this peptide named as 1-36).

The listed protein antigen for TID can be specific to dog IAPP. The listed epitope peptide from dog IAPP refer to sequence 10 (this peptide named as 1-36).

The disclosed protein antigen for TID can be specific to cat IAPP. The disclosed epitope peptide from cat IAPP refer to sequence 11 (this peptide named as 1-36).

In which, sequence 1 is composed by 15 amino acids; sequence 2 is composed by 9 amino acids; sequence 3 is composed by 31 amino acids; sequence 4 is composed by 15 amino acids; sequence 5 is composed by 9 amino acids; sequence 6 is composed by 15 amino acids; sequence 7 is composed by 9 amino acids; sequence 8 is composed by 10 amino acids; sequence 9 is composed by 37 amino acids; sequence 10 is composed by 37 amino acids; sequence 11 is composed by 37 amino acids; sequence 12 is composed by 17 amino acids; sequence 13 is composed by 37 amino acids;

The compositions of protein antigen and immune suppressive agents listed in 1) are disclosed to have the following ratios (quantity ratio) such as 1:20 to 20:1, 1:1 to 10:1. The specific ratios are 1:1 (10µg protein antigen + 10µg immune suppressive agent) or 10:1 (10µg protein antigen + 1µg immune suppressive agent)

The compositions of epitope peptide antigen and immune suppressive agents listed in 2) can be made as 1g: 1g.

At least one function of provided compositions in this invention listed below:
(1) Treatment or prevention of TID for vertebrate;
(2) Enhancing the proliferation of CD4⁺CD25⁺Treg population in mammalian;
(3) Increasing the ratio of CD4⁺CD25⁺Treg to CD4⁺T cells in mammalian;
(4) Increasing the IL-10 secretion from T cells in mammalian;
(5) Inhibiting the cytotoxicity effect of auto-reactive CD8⁺T cells in mammalian; The described inhibiting the cytotoxicity effect of CD8⁺T cells refer to cytotoxicity against islet cells or /and spleen cells.
(6) Controlling the blood glucose for TID individuals(mammalian);
(7) Up-regulating the transcription level of IL-10 or/and TGF-β in PBMC or/ and spleen;
(8) Inhibiting the DC maturation; The described inhibiting the DC maturation refer to down-regulating at least one of surface proteins (CD40, CD80, CD83, CD86) in DC;

The listed vertebrates are mammalian that can be specific to mouse, cat, dog or human.

Products of these compositions for the use in the treatment or/and prevention of TID listed in invention, are also protected by this invention

The protection area in this invention is listed in below a)- h) product areas when the compositions described in this invention are used for the treatment or/ and preventionof T1D meeting at least one application below.
a) Increasing the ratio of CD4⁺CD25⁺Treg and CD4⁺T cells in mammalian;
b) Enhancing the proliferation of CD4⁺CD25⁺ Treg population in mammalian;
c) Increasing the IL-10 secretion from T cells in mammalian;
d) Controlling the blood glucose for TID in mammalian;
e) Inhibiting the cytotoxicity effect of auto-reactive CD8⁺T cells;
f) Up-regulating the transcription level of IL-10 or /and TGF-β in PBMC or/ and spleen;
g) Inhibiting the DC maturation in mammalian;
h) Decreasing the secretion of at least one of surface proteins (CD40, CD80, CD83, CD86) in DC;

The listed vertebrates are mammalian that can be specific to mouse, cat, dog or human.

The compositions for use in the treatment or/and prevention of TID listed in invention, are also protected by this invention

The drug compositions and their use according to the invention can inhibit TID by inducing CD4⁺CD25⁺ Treg after administration of compositions listed in invention.

The drug compositions listed in invention can be made as following ratios when applied, 1) Protein antigen for TID, the concentration of insulin is disclosed to be 0.01∼1IU per kg of body weight, e.g. 0.15∼0.25IU per kg body weight, the concentration of immune suppressive agent Dex is disclosed to be 0.01∼600µg per kg body weight, e.g. 1∼5µg per kg body weight;
2) As the TID epitope peptide antigen, the concentration of B23-29 is disclosed to be 0.05∼1µg per kg of body weight, e.g. 0.05∼1µg per kg body weight, the concentration of immune suppressive agent Dex is disclosed to be 0.01∼1µg per kg body weight, e.g. 0.05∼0.2µg per kg body weight;
3) As the TID epitope peptide antigen, the concentration of B10-C5 is disclosed to be 0.05∼1µg per kg of body weight, e.g. 0.05∼1µg per kg body weight, the concentration of immune suppressive agent Dex is disclosed to be 0.01∼1µg per Kg body weight, eg. 0.05∼0.2µg per Kg body weight;

The compositions and their use for treatment or/and prevention of TID in this invention can be delivered by injection, ejection, instillation, infiltration, absorption or physical and chemically delivered by im, id, sc, iv, intra mucosal; or delivered by combining with other substrate mixed or packaged.

The compositions can be administrated every 3-30 days and total 4-8 administrations.

### Figure Legends

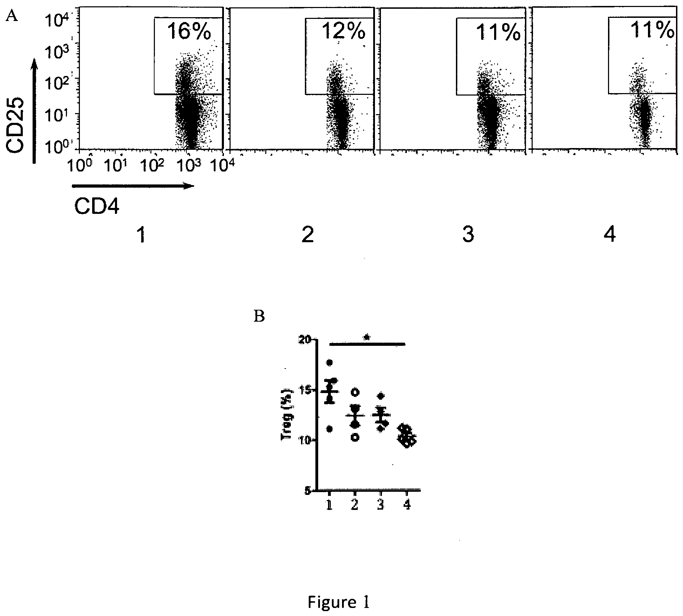

**Figure 1****.** The dose effects of compositions and methods on Treg population. The NOD mice were immunized with different doses of genetic engineered recombinant human insulin (rh-insulin) and DEX, the percentage of CD4⁺CD25⁺Treg was measured in spleen. A. FACS result; B. Statistical analysis of FACs data. In Fig A and B, 1 refer to group1 (10 + 10) in which 10µg rh-insulin and 10 µg DEX mixed treatment; 2 refer to group 2 (100 + 100) in which 100µg rh-insulin and 100 µg DEX mixed treatment; 3 refer to group 3 (500 + 100) in which 500µg rh-insulin and 100 µg DEX mixed treatment; 4 refer to group 4 in which the NOD mice without treament.

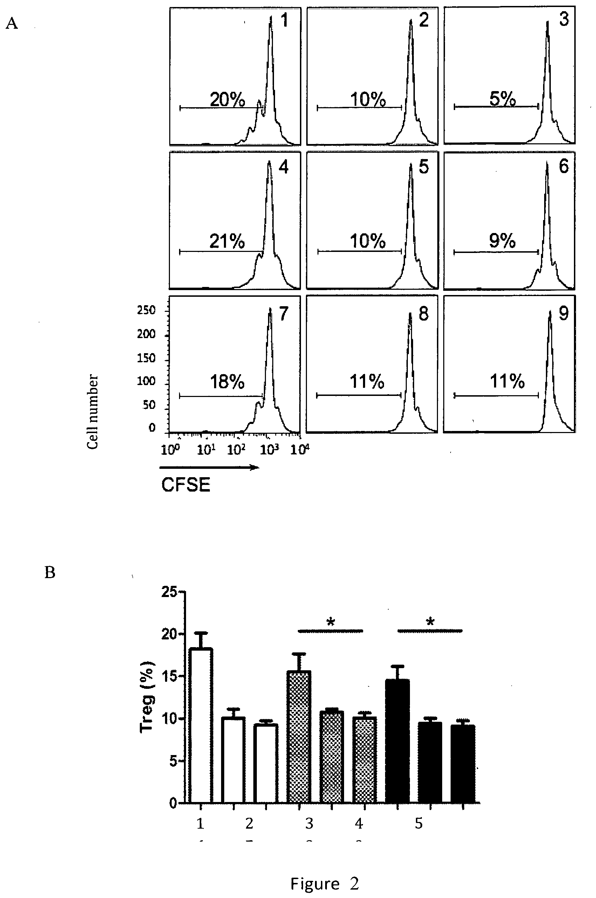

**Figure 2****.** The dose effect of compositions and methods on Treg proliferation. The NOD mice were immunized with different doses of rh-insulin and DEX. The purified CD4⁺CD25⁺Treg from spleen were labeled with CFSE and stimulated with rh-insulin or human insulin epitope peptide hB9-23 in vitro. After stimulation, the proliferation of Treg was measured. A. FACS result; B. Statistical analysis of FACs data. In Fig A and B, 1 refer to positive control in which the cell treated with anti-CD3 antibody; 2 refer to non-related control in which the cell treated with OVA323-339; 3 refer to negative control (The cells tested in 1,2,3 are from unimmunized NOD mice); 4 refer to stimulated group in which the cell stimulated with 10µg rh-insulin and 10 µg DEX mixture; 5 refer to stimulated group in which the cell stimulated with 100µg rh-insulin and 100µg DEX mixture; 6 refer to stimulated group in which the cell stimulated with 500µg rh-insulin and 100 µg DEX mixture; 7 refer to stimulated group in which the cell stimulated with 10µg human insulin epitope peptide B9-23 and 10 µg DEX mixture; 8 refer to stimulated group in which the cell stimulated 100µg human insulin epitope peptide B9-23 and 100 µg DEX mixture; 9 refer to stimulated group in which the cell stimulated 500µg human insulin epitope peptide B9-23 and 100 µg DEX mixture;

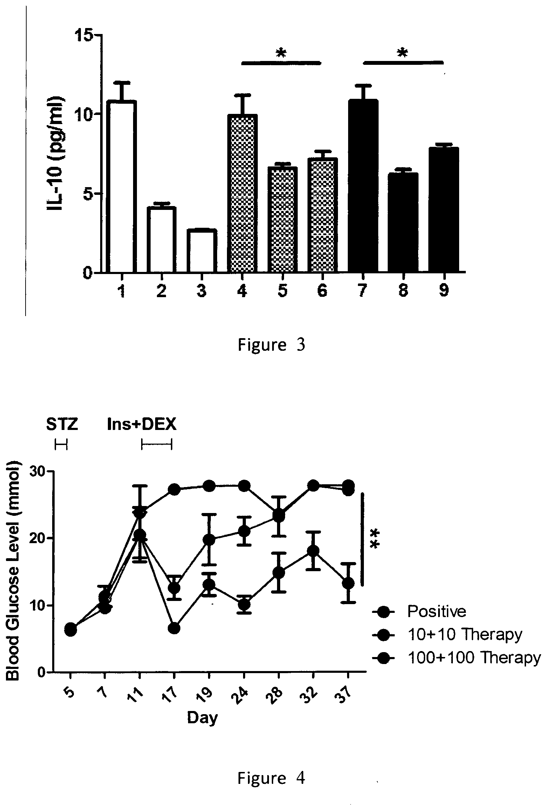

**Figure 3****.** The dose effects of compositions and methods on expression of IL-10. The NOD mice were immunized with different doses of rh-insulin and DEX. The purified CD4⁺ CD25⁺Treg from spleen was stimulated with rh-insulin or human insulin epitope peptide hB9-23 in vitro. After stimulation, the level of IL-10 from supernatant was measured (pg/ml). 1 refer to positive control in which the cell treated with anti-CD3 antibody; 2 refer to non-related control in which the cell treated with OVA323-339; 3 refer to negative control (The cells tested in 1,2,3 are from unimmunized NOD mice); 4 refer to stimulated group in which the cell stimulated with 10µg rh-insulin and 10 µg DEX mixture; 5 refer to stimulated group in which the cell stimulated with 100µg rh-insulin and 100µg DEX mixture; 6 refer to stimulated group in which the cell stimulated with 500µg rh-insulin and 100 µg DEX mixture; 7 refer to stimulated group in which the cell stimulated with 10µg human insulin epitope peptide B9-23 and 10 µg DEX mixture; 8 refer to stimulated group in which the cell stimulated 100µg human insulin epitope peptide B9-23 and 100 µg DEX mixture; 9 refer to stimulated group in which the cell stimulated 500µg human insulin epitope peptide B9-23 and 100 µg DEX mixture;
**Figure 4****.** The blood glucose level in acute TID after treatment with compositions and methods. The change of blood glucose (mM) was measured in NOD mice induced by STZ with clinic sign of TID after treated with high dose (100µg rh-insulin and 100µg DEX) and low dose (10µg rh-insulin and 10µg DEX). Empty circle with solid line refer to the TID group without treatment; Filled circle with solid line refer to the TID group with low dose treatment (10 + 10); Empty circle with dash line refer to the TID group with high dose treatment (100 + 100);

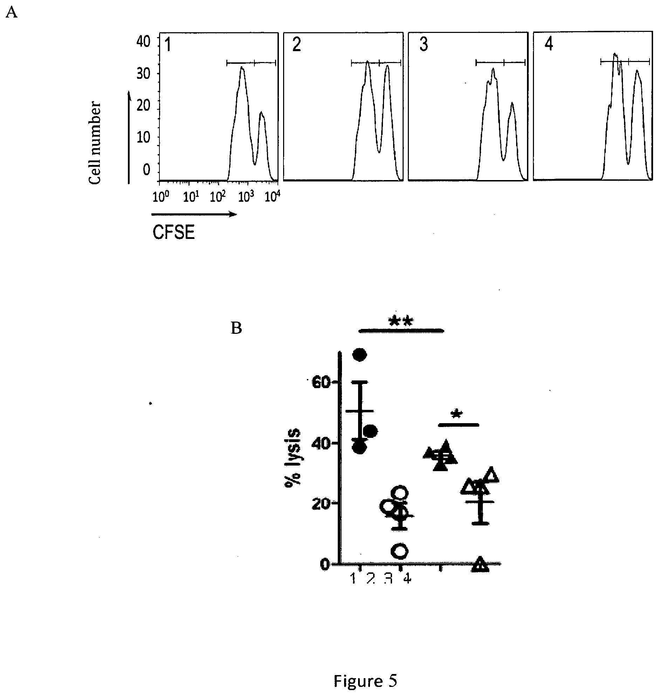

**Figure 5****.** The CTL response in acute TID after treatment with compositions and methods. The CTL response was measured in NOD mice induced by STZ with clinic sign of TID after treated with high dose (100µg rh-insulin and 100 µg DEX) and low dose (10µg rh-insulin and 10µg DEX). The CFSE labeled spleen cells were incubated with CD8 T cell epitope peptide of insulin 10-18 and were transferred into the NOD mice with treatment. The CTL response was measured 12 hour later of transfer. A. FACS result; B. Statistical analysis of FACs data. In Fig A and B, 1 refer to group1 CTL result in which the NOD mice show clinical sign; 2 refer to group 2 (10 + 10) in which 10µg rh-insulin and 10 µg DEX mixed treatment; 3 refer to group 3 (100 + 100) in which 100µg rh-insulin and 100 µg DEX mixed treatment; 4 refer to group 3 (100 + 100) in which the NOD mice pre-injected with anti-CD8 antibody were immunized with 100µg rh-insulin and 100 µg DEX.

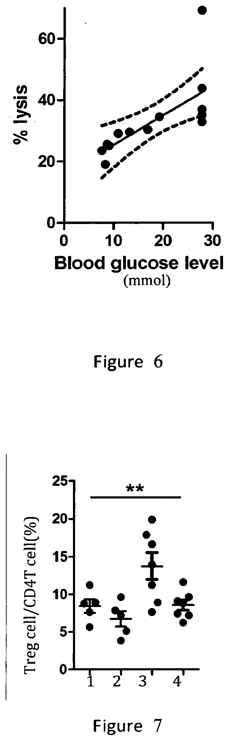

**Figure 6****.** The CTL response relationship between levels of blood glucose and auto-reactive CTL in acute TID after treatment. In which each dot refer to single mouse. CTL response in TID mice after treatment listed compositions.
**Figure 7****.** The level of Treg (Foxp3⁺CD4⁺) in acute TID after treatment. The ratio of Treg (Foxp3⁺CD4⁺) with CD4+ T cells was measured in NOD mice induced by STZ with clinic sign of TID after treated with high dose (100µg rh-insulin and 100µg DEX) and low dose (10µg rh-insulin and 10µg DEX). 1 refer to control group in which the NOD mice show no clinical sign and no treatment; 2 refer to group 1 in which the NOD mice show clinical sign; 3 refer to group 2 (10 + 10) in which 10µg rh-insulin and 10 µg DEX mixed treatment; 4 refer to group 3 (100 + 100) in which 100µg rh-insulin and 100 µg DEX mixed treatment.

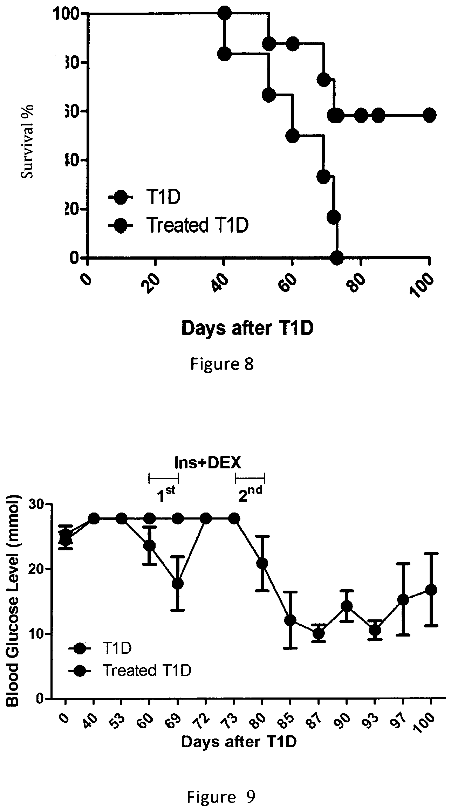

**Figure 8****.** The survival curve of long term TID after treatment. When the NOD mice show clinic sign of TID, the survive rate was recorded timely after NOD mice with TID were treated with low dose (10µg rh-insulin and 10µg DEX). Empty circle with solid line refer to group 1 (Diabetic group without treatment); Filled circle with solid line refer to group 2 (Treatment group).
**Figure 9****.** The level of blood glucose change result of long term TID after treatment. After NOD mice were induced by STZ with clinic sign of TID, the level of blood glucose was measured after the mice were treated with low dose (10µg rh-insulin and 10µg DEX). Empty circle with solid line refer to group 1 (Diabetic group without treatment); Filled circle with solid line refer to group 2 (Treatment group);

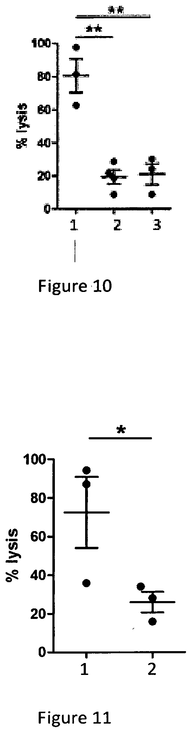

**Figure 10****.** The CTL response against insulin10-18 in long term TID. After NOD mice were induced by STZ with clinic sign of TID, the mice were treated with low dose (10µg rh-insulin and 10µg DEX). The CFSE labeled cells were incubated with CD8 T cell epitope peptide of insulin10-18 and were transferred into the NOD mice with treatment. The CTL response was measured 12 hour later of transfer. 1 refer to CTL result of group 1(Diabetic group without treatment);; 2 refer to CTL result of group 2 (Treatment group) in which 10µg rh-insulin and 10 µg DEX mixed treatment; 3 refer to group 3 in which the CTL were blocked with anti-CD8 mAb.
**Figure 11****.** The CTL response against islet cells in long term TID. After NOD mice were induced by STZ with clinic sign of TID, the mice were treated with low dose (10µg rh-insulin and 10µg DEX). The CFSE labeled islet cells were transferred into the NOD mice with treatment. The CTL response was measured 12 hour later of transfer. 1 refer to CTL result of group 1(Diabetic group without treatment);; 2 refer to CTL result of group 2 (Treatment group) in which 10µg rh-insulin and 10 µg DEX mixed treatment;

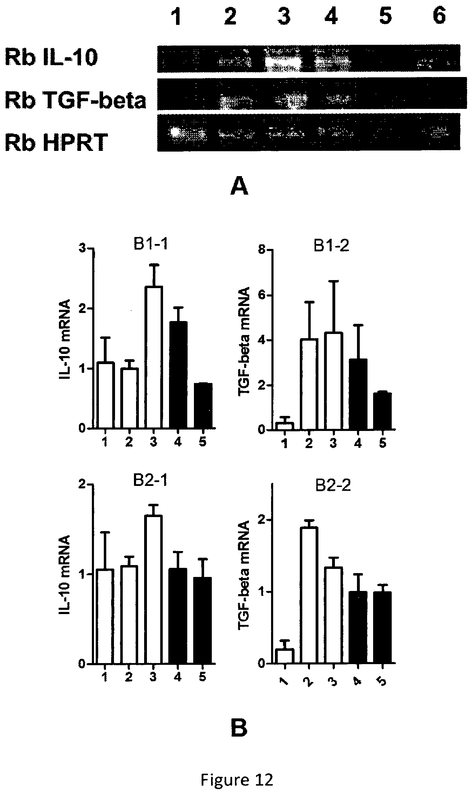

**Figure 12****.** The dose effects of the treatment on expressions of IL-10 and TGF-β in rabbits. After the rabbits were treated with different doses of rh-insulin and DEX. The spleens were isolated and splenocytes were stimulated with rh-insulin in vitro. After stimulation, the level of IL-10 and TGF-β were measured by RT-PCR. A, result of RT-PCR electrophoresis from PBMC, lane 1 refer to group 1 (100 + 100) in which 100µg rh-insulin and 100 µg DEX mixed treatment; lane 2 refer to group 2 (10 + 10) in which 10µg rh-insulin and 10 µg DEX mixed treatment; lane 3 refer to group 3 (10 + 1) in which 10µg rh-insulin and 1 µg DEX mixed treatment; lane 4 refer to group 4 (DEX100) in which 100 µg DEX treatment; lane 5 refer to group 5 (Ins100) in which 100µg rh-insulin treatment; lane 6 refer to group 6 (Negative control) in which 100ul PBS treatment. B, result of RT-PCR from PBMC and spleen, B1-1 refer to RT-PCR of IL-10 from PBMC; B1-2 refer to RT-PCR of TGF-β from PBMC; B2-1 refer to RT-PCR of IL-10 from spleen; B2-2 refer to RT-PCR of TGF-β from spleen; In B1-1, B1-2, B2-1 and B2-2, lane 1 refer to group 1 (100 + 100) in which 100µg rh-insulin and 100 µg DEX mixed treatment; lane 2 refer to group 2 (10 + 10) in which 10µg rh-insulin and 10 µg DEX mixed treatment; lane 3 refer to group 3 (10 + 1) in which 10µg rh-insulin and 1 µg DEX mixed treatment; lane 4 refer to group 4 (DEX100) in which 100 µg DEX treatment; lane 5 refer to group 5 (Ins100) in which 100µg rh-insulin treatment.

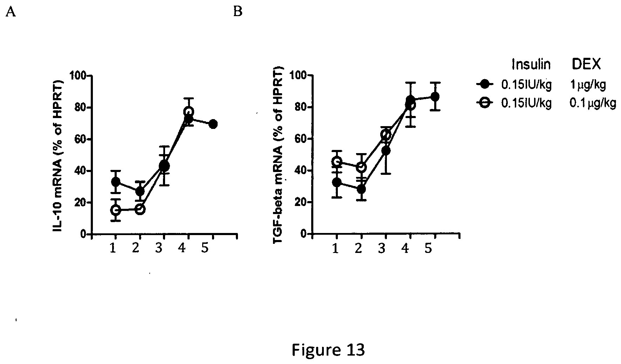

**Figure 13****.** The dose effect of treatment on expressions of IL-10 and TGF-β in dogs. After TID dog were treated with different doses of rh-insulin and DEX. Their splenocytes was isolated at days -3, 0, 8, 20, 28 and stimulated with rh-insulin in vitro. The level of IL-10 and TGF-β were measured by RT-PCR. A, Result of IL-10 levels, B, Result of TGF-β levels. The 1, 2, 3, 4, 5 in A and B stand for RT-PCR result of sample on days - 3, 0, 8, 20, 28.

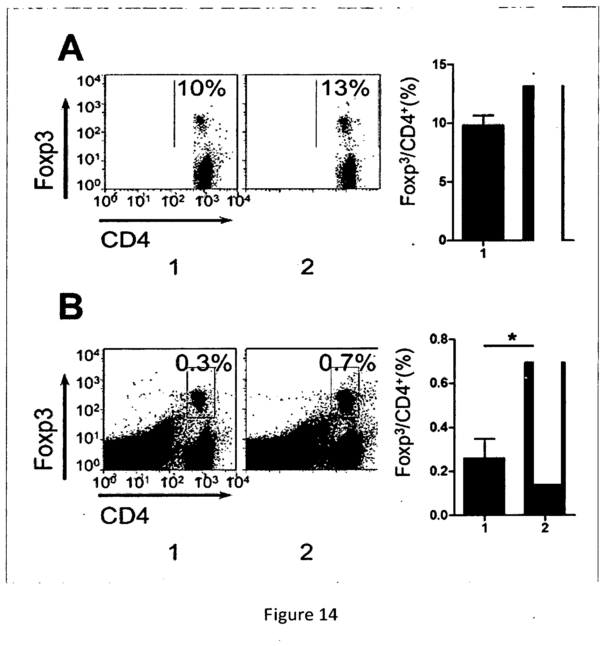

**Figure 14****.** The effect of the treatment on Treg cell population in acute TID dogs. The Alloxan induced TID dogs were treated with rh-insulin and DEX, the percentage of CD4⁺CD25⁺Treg among all cells in pancreas was measured. A, Ratio of Treg to CD4+ T cells; B, Ratio of Treg to all cells; In A and B, 1, refer to diabetic animals; 2, refer to group treated with 100µg rh-insulin and 1.5 µg DEX.

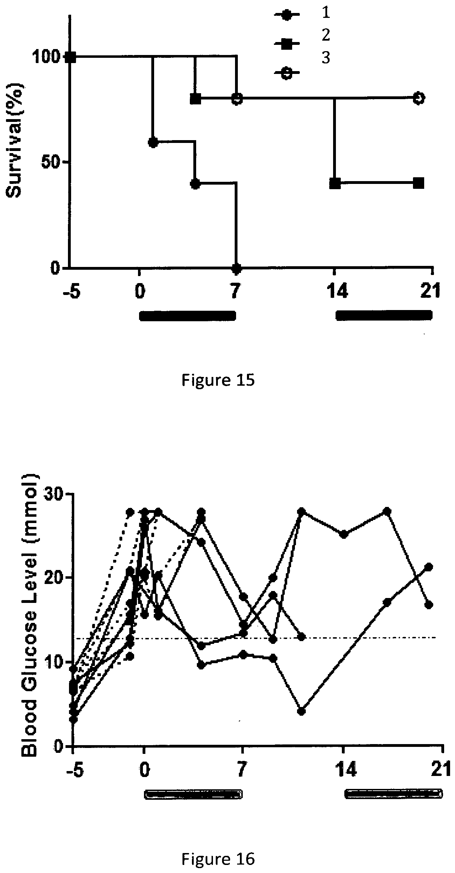

**Figure 15****.** The survival of TID dogs after the treatments. After TID dogs were treated with rh-insulin and DEX or rh-insulin and CsA, their survival was followed. 1 refer to dogs treated with rh-insulin at 0.15IU/kg body weight and DEX at 1ug/Kg body weight; 2 refer to dogs treated with rh-insulin at 0.15IU/kg body weight and CsA at 100ug/Kg body weight; 3 refer to diabetic model control. The grey line indicates treatment duration, in which 3 injections applied to each cycle of treatment.
**Figure 16****.** TID dog blood glucose level change after the treatments. After TID dogs were treated with rh-insulin and DEX, the level of blood glucose was followed. Solid line refers to dogs treated with rh-insulin at 0.15IU/kg body weight and DEX at 1ug/Kg body weight; Dashed line refers to diabetic model control. The grey line indicates treatment duration, in which 3 injections applied to each cycle of treatment. Vertical line is for defining the high level of blood glucose.

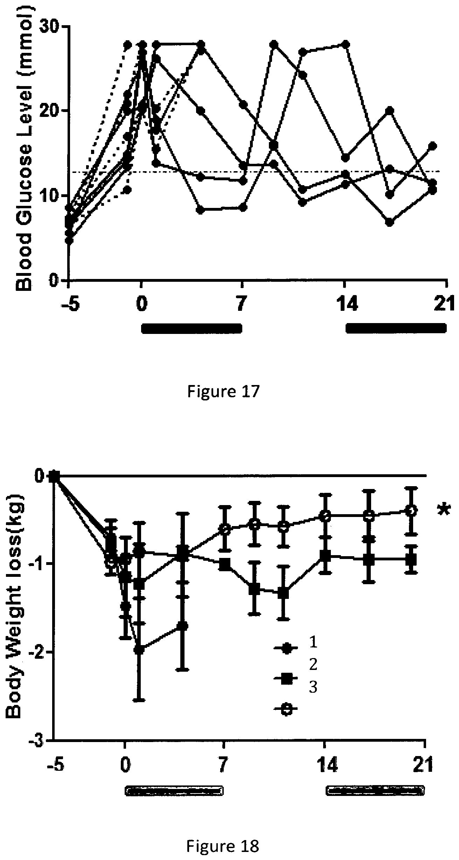

**Figure 17****.** TID dog blood glucose level change after the treatments. After TID dogs were treated with rh-insulin and CsA, the level of blood glucose was followed. Solid line refers to dogs treated with rh-insulin at 0.15IU/kg body weight and CsA at 100ug/Kg body weight; Dash line refers to diabetic model control. The grey line indicates treatment duration, in which 3 injections applied to each cycle of treatment. Vertical line is for defining the high level of blood glucose.
**Figure 18****. TID** dog body weight loss after the treatments. After TID dogs were treated with rh-insulin and DEX, their body weight changes were followed. 1 refer to dogs treated with rh-insulin at 0.15IU/kg body weight and Dex at 1ug/Kg body weight; 2 refer to dogs treated with rh-insulin at 0.15IU/kg body weight and CsA at 100ug/Kg body weight; 3 refer to diabetic model control. The grey line indicates treatment duration, in which 3 injections applied to each cycle of treatment.

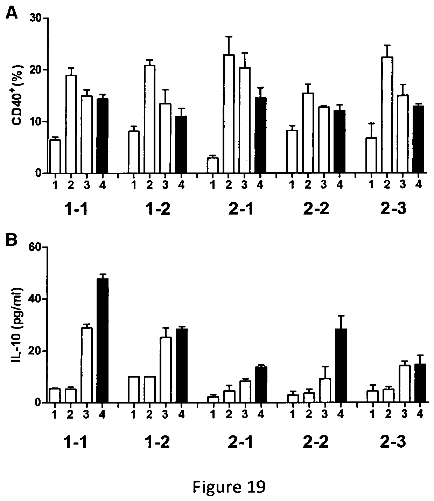

**Figure 19****.** The expression of CD40 and IL-10 in DC converted from PBMC. The isolated PBMC from normal people and TID patients were induced into CD1a⁺ DC by GM-CSF and IL-4. Afte CD1a⁺ DCs were stimulated with insulin and DEX, the expression of CD40 and IL-10 were measured. In which, A refer to the result of CD40 expression; B refer to the result of IL-10 expression. The 1-1 and 1-2 in A and B refer to result of 2 TID patient; the 2-1, 2-2 and 2-3 in A and B refer to result of 3 normal human samples; In 1-1, 1-2, 2-1, 2-2 and 2-3, 1, refer to negative control; 2 refer to samples treated with rh-insulin at final concentration of 10µg/ml; 3 refer to samples treated with DEX at final concentration of 10µg/ml; 4 refer to samples treated with rh-insulin and DEX at final concentration of 10µg/ml.

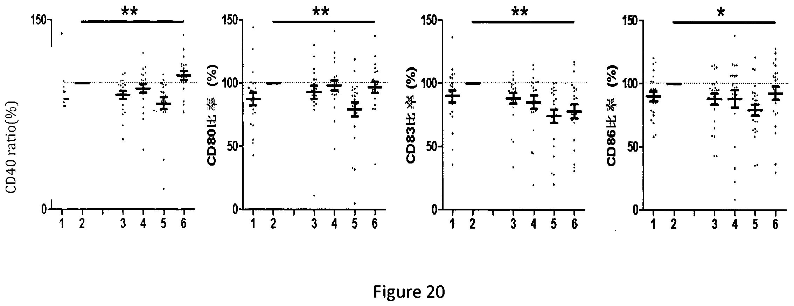

**Figure 20****.** The high throughput screening results from massive samples for the compositions and methods. The expression profile of CD40, CD80, CD83 and CD86 on DC after human PBMC treated with compositions. 1 refer to negative control; 2 refer to samples treated with rh-insulin at final concentration of 10µg/ml; 3 refer to samples treated with both rh-insulin and DEX at final concentration of 10µg/ml; 4 refer to samples treated with both rh-insulin and Rap at final concentration of 10µg/ml; 5 refer to samples treated with both rh-insulin and CsA at final concentration of 10µg/ml; 6 refer to samples treated with both rh-insulin and FK506 at final concentration of 10µg/ml; Each dot stands for single blood sample.

### Example of Experiments:

The regular procedure applied if no specific statements addressed in following examples.

All materials and reagents in following procedure are commercially available if no specific statement addressed.

Sources of compositions for the treatments: The rh-insulin was from Novo Nordisk A/S; 1IU rh-insulin equal to 45.4µg in quantity. The peptides of insulin were synthesized by Beijing Aoke in which the B9-23 peptide from sequence 1 refer to epitope peptide for TID; All immune-suppressors were GMP grade and produced by China Pharma Group. In which DEX is H34023626, Rap is SR039501, FK506 is H20080457 and CsA is H10940045. Compositions were made by protein, or peptide mixed with immune-suppressors before injection.

### Example 1. Dose effect of compositions and methods on NOD mice.

### Immunizations of NOD mice

### 1. Immunization with compositions of rh-insulin and DEX.

The NOD mice were divided into 4 groups with 3 mice per group. The drug compositions of rh-insulin and DEX were administrated into each group from s.c on days 1, 4 and 7. Group 1(10 + 10) was injected with 10µg of rh-insulin and 10µg of DEX dissolved in 100 ul of PBS. Group 2 (100 + 100) was injected with 100µg of rh-insulin and 100µg of DEX dissolved in 100 ul of PBS. Group 3 (500 + 100) was injected with 500µg of rh-insulin and 100µg of DEX dissolved in 100 ul of PBS. All animals were injected on days 1, 4 and 7.

### 2. Immunization with compositions of insulin epitope peptides B9-23 and DEX.

The NOD mice were divided into 4 groups with 3 mice per group. The drug compositions and methods were delivered into each group from s.c on day 1, 4, 7. Group 1 (10 + 10) was injected with 10µg of human insulin epitope peptides B9-23 and 10µg of DEX dissolved in 100 ul of PBS. Group 2 (100 + 100) was injected with 100µg of human insulin epitope peptides B9-23 and 100µg of DEX dissolved in 100 ul of PBS. Group 3 (500 + 100) was injected with 500µg of human insulin epitope peptides hB9-23 and 100µg of DEX dissolved in 100 ul of PBS. All animals were injected days 1, 4 and 7.

### Determination of dose effect of drug compositions and methods by measuring the population and proliferation of Treg and IL-10 expression.

### 1. The dose effect of drug compositions and methods on Treg population.

The drug effect of suppressive was determined with percentage of Treg population in immunized NOD mice at day 8 after the last immunization.

Detail procedure listed below:
1) The spleen was taken out and placed into dish containing 2ml of RPMI-1640 under standard sterilization protocol.
2) The spleen was placed on sterilized bronze container and smashed by front end of syringe.
3) The single suspension was filtered into 15 ml tube and spin down at 2000rpm for 10 minutes.
4) The supernatant was discarded, 2-3ml red blood cell lysis buffer added into the cell pellet and react for 2 min before stopped by adding equal volume of RPMI-1640. Spin down at 2000rpm for 10 minutes.
5) The supernatant was discarded, 3-4 ml of RPMI-1640 with 2% of FBS was added and the cell pellet was re-suspended.
6) The cell solution was filtered by glass fiber column to remove the B cells.
7) Counted the cell density.
8) Wash the cell once with PBS and adjust the cell density at 2×10⁷
9) 10⁶ cells were stained with CD4 and CD25 mAb by adding 0.2µl of PE-anti-CD4 mAb and 0.2µl of APC-anti-CD25 (eBioscience 12-0041, 17-0251) at RT for 10 minutes along with light avoiding. The percentage of Treg was measured by FACs compared with cells from NOD mice without immunization.

The result in Figure 1 shows the group 1 (10 + 10) injected with 10µg of rh-insulin and 10µg of DEX increase Treg frequency upto 16%, whereas only 10-12% of Treg maintained in other groups.

### 2. The dose effect of drug compositions and methods by detecting Treg proliferation.

The drug effect was determined with proliferation of Treg against insulin (rh-insulin and DEX) and B9-23 (human insulin epitope peptide B9-23 and DEX) in immunized NOD mice at day 8 after the last immunization. In which, the T cells were stained with CFSE and the proliferation of Treg was measured by FACs.

Detail procedure listed below:
Steps 1-9 are same as method mentioned above whereas 10) the spleen cells were stained with 3µM CFSE at room temperature (RT) for 8 minutes with shaking in step 100.
11) Adding equal volume of FBS to stop the staining reaction. Spin down and wash the cells for 3 times.
12) Add the cells into 96-well plate and each well add 2×10⁵ cells. Then 100µl Anti-CD3 mAb (AbDSerotec, MCA500GA) with final concentration at 1µg/ml were added into one well as positive control, one well was added with 5µg/ml of OVA323-339 (ISQAVHAAHAEINEAGR) as the un-related control. One well was added with 10µg/ml of human insulin epitope peptides hB9-23 to stimulate cell proliferate. Also the cells without CFSE staining were added into the well as the control.
13) Incubate the cells at 37°C, 5% CO₂ for 3 days. FACs measured the proliferation of Treg in comparison with untreated and non-diseased NOD mice.

The result in Figure 2 shows that group 1 (10 + 10) injected with 10µg of rh-insulin plus 10µg of DEX or 10µg of human insulin epitope peptides hB9-23 plus 10µg of DEX significantly enhanced Treg proliferation comparing with other groups.

### 3. Dose effect of drug compositions and methods by detecting IL-10 expression in Treg.

The drug effect was determined by IL-10 expression of Treg in NOD mice immunized with 10µg of rh-insulin plus 10µg of DEX and 10µg of human insulin epitope peptides B9-23 plus 10µg of DEX at day 8 after the last immunization.

Detail procedure listed below:
Steps 1-9 are same as method mentioned above.
10) Add the cells into 96-well plate and each well add 2×10⁵ cells. Then 100µl Anti-CD3 mAb with final concentration at 1µg/ml were added into one well as positive control, one well was added with 5µg/ml of OVA323-339 as the un-related control. One well was added with 10µg/ml of human insulin epitope peptides hB9-23 to stimulate cell. One well was added with 10µg/ml of rh-insulin to stimulate cell. Also the cells without stimulation were added into the well as the negative control.
11) Incubate the cells at 37°C, 5% CO₂ for 24 hours. The 30ul supernatant were collected and mixed with 30 ul PBS which contained 0.1µl FlexSet microbead (BD, 558300) for 30 minutes. The 30 ul PBS that contained 0.1µl PE labeled antibody were added and incubated for another 30minutes. FACs measured the IL-10 expression in comparison with naive NOD mice.

The result in Figure 3 shows that group 1 (10 + 10) injected with 10µg of rh-insulin plus 10µg of DEX or 10µg of human insulin epitope peptides hB9-23 plus 10µg of DEX significantly increased the expression of IL-10.

In summary, the results from this example show that rh-insulin plus DEX, or human insulin epitope peptides plus DEX can induce Treg production in NOD mice. This Treg can be proliferated and can produce IL-10 caused by human insulin and human insulin epitope peitide B9-23. The best dose for this effect is 10µg of rh-insulin plus 10µg of DEX and 10µg of human insulin epitope peptides B9-23 plus 10µg of DEX

### Example 2. Treatment effect of drug compositions and methods on acute TID NOD mice.

### NOD mice induction and immunizations

After the dose of drugs determined in example 1, the rh-insulin plus DEX were inject into diabetic NOD mice of which levels of blood glucose were more than 12 mmol by i.p.

For establishment of TID model, 18 NOD mice were induced by i.p. injections with 40mg/kg STZ (Sigma Aldridge, S0130) for 5 continuous days. When the level of blood glucose reached higher than 12mmol, those NOD mice were divided into 3 groups with 6 mice per group. The group 1 (diabetic group) was un-treatment group. Group 2 (10 + 10) was treated with 10µg of rh-insulin plus 10µg of DEX. Group 3 (100 + 100) was treated with 100µg of rh-insulin plus 100µg of DEX. The drugs were injected from i.p. on days 1, 4 and 7 after the mice were determined as TID mice.

### Detection of lood glucose levels change, CTL and Treg.

### 1. Detection of blood glucose levels change.

The blood glucose was monitored on days 5, 7,11, 17, 19,24, 28, 32 and 37 to reflect the drug effect after injection

Test procedure: 10ul of blood samples were dropped to blood glucose test strips and a test instrument read the concentration of blood glucose.

The result in Figure 4 shows that group 2 (10+10) injected with 10µg of rh-insulin plus 10µg of DEX can control the level of blood glucose at 10-12 mmol whereas group 3 (100+100) injected with 100µg of rh-insulin plus 100µg of DEX show decreasing glucose level at the beginning of treatment, but increased at the same level as diabetic group finally.

### 2. Detection of CTL response.

The induced Treg cells could suppress auto-reactive CD8 T cell responses after drug injections. The CTL responses were detected on day 37.

Detail procedure listed below:
1) The CD8 T cells were depleted by injection of anti-CD8 mAb (eBioscience, clone 53-6.7) on day 35 and 36 for mice immunized with 100µg of rh-insulin plus 100µg of DEX.
2) The spleen cells from normal untreated NOD mice were isolated and counted, the methods are same as mentioned above 1)-9).
3) The equal number of cells was stained with 5µM and 20µM of CFSE for 8 minutes at RT. The staining stopped by adding equal volume of FBS followed by 3 time washes.
4) The cells stained with 20µM of CFSE were incubated with 50µg/ml of Insulin10-18 CD8 T epitope peptide (HLVEALYLV) at 37°C, 5% CO₂ for 30min followed by wash.
5) Mix equal number of cells stained with 5µM and 20µM of CFSE and adopt transfer the mixed cell into NOD mice described in step 1.
6) The spleen cell were isolated after 12 hours' transfer, the specific lysis of target was measured by FACs and formula (Specific lysis ratio= 1-target cells/control cells×100%,) was applied.

The result in Figure 5 shows that group 2 (10+10) injected with 10µg of rh-insulin plus 10µg of DEX can significantly suppress the CTL responses from auto-reactive CD8 T cells, whereas the CTL responses from the group injected with 100µg of rh-insulin plus 100µg of DEX showed less degree of the suppression. This CTL response can be blocked by CD8 antibody, which was apparently correlated with the level of blood glucose and thus demonstrated that the controlled blood glucose was due to the improved autoimmune activity.

### 3. Detection of mouse Treg population.

The Treg cells can be induced by drug treatments, thus the Treg population was detected on day 37. Detail procedures are same as part 2 of example 1.

The result in Figure 7 shows that group 2 (10+10) injected with 10µg of rh-insulin plus 10µg of DEX can increase Treg frequency to 15%, whereas only 8-10% of Treg in other groups.

In summary, the result from this example shows that rh-insulin plus DEX can induce Treg production in NOD mice and this Treg population can be explained that cease of CTL activities and islet attacking from the auto-reactive CD8 T cells by such induction of Treg cells.

### Example 3. Effect of drug compositions and methods on long-term TID NOD mice.

The example 2 demonstrated that the drug compositions possess the effect against short term TID. The following example shows the long-term effect of the drug treatment.

### NOD mice induction and immunizations

For establishment of TID model, 16 NOD mice were induced by i.p. injection of 40mg/kg STZ for 5 continuous days. After 2 month NOD mice showed the diabetic symptom, animals were divided into 2 groups with 8 mice per group. The group 1 (diabetic group) was un-treatment group. Group 2 (treatment group) was treated with 10µg of rh-insulin plus 10µg of DEX. The drug was injected from i.p on days 1, 4 and 7 as one treatment cycle. A second treatment cycle was used one week later after the first cycle.

### Detection of survival, blood glucose levels change and CTL.

### 1. Survival of TID NOD mice.

The survival was observed timely for 100 days after treatments of TID NOD mice.

The result in Figure 8 shows that injections with rh-insulin plus DEX can make 60% of TID NOD mice survived on day 100, whereas all animals dead on days 60 to 80 in the untreated group.

### 2. Blood glucose levels change detection.

The levels of blood glucose were monitored on days 0, 40, 53, 60, 69, 72, 73, 80, 85, 87, 90, 93, 97 and 100 to reflect the drug effect.

Test procedure: 10 ul of blood samples were spotted onto the test strips and the test instrument read the concentration of blood glucose.

The result in Figure 9 shows that group 2 injections with rh-insulin plus DEX can control the level of blood glucose at 10-15 mmol, whereas no control in the control group 1.

### 3. Mice CTL response detection.

The CTL response was detected on day 60 for the control group and on day 100 for the treatment group.

Detail procedures are same as part 2 of example 2.

Additionally, the CTL response against islet cell was measured. In which the pancreatic cell isolated from naive NOD mice used as target cell. The specific procedures are: 1) The NOD mice were sacrificed and the pancreatic tissues and liver exposed under sterilized condition. 2) The 10ml 1mg/ml of Collagenase P(Roche, Cat. No. 11213857001) were injected into pancreatic tissue and digested for 1 hour at 37°C. 3) The pancreatic cells were washed twice and spin down at 250g for 1 minute. 4) The cell pellets were re-suspended with 3ml of 25% Ficoll (Roche) and in turns 2ml of 23% Ficoll, 2ml of 20% Ficoll, 2ml of 11% Ficoll were added into above the 23% Ficoll. Then spin down the cell solution at 800g for 10 minutes and the islet cell phase were taken out, the Ficoll solution in islet cell was washed out with twice PBS washing. 5) The islets were digested with 0.25% of trypsin for for 10 minutes at 37°C. 6) The single suspension islet cells were stained with 20 uM CFSE as the target cells whereas the effect cells stained with 5uM CFSE were added into the target cells.

The result shows that the auto-reactive CD8 T cells against insulin 10-18 were significantly inhibited in the treated group, but not in the control group. The inhibition effect can be blocked by anti-CD8 mAb (Fig 10). Similarly, the CTL response against islet cells was also controlled after the treatments (Fig 11).

### Example 4. Dose effect of drug compositions in rabbits.

It demonstrated in example 1-3 that rh-insulin and DEX have therapeutic effect against TID in NOD mice, and then the effect was evaluated in rabbit.

### Immunization of rabbits

18 rabbits were divided into 6 groups with 3 rabbits per group. The drug was injected i.p on days 1, 4 and 7 as one treatment cycle. The group 1 (100 + 100) was treated with 100µg of rh-insulin plus 100µg of DEX. Group 2 (10 + 10) was treated with 10µg of rh-insulin plus 10µg of DEX. Group 3 (10 + 1) was treated with 10µg of rh-insulin plus 1µg of DEX. Group 4 (DEX100) was treated with 100µg of DEX alone. Group 5 (Ins100) was treated with 10µg of rh-insulin alone. Group 6 (negative control) was treated with 100ul of PBS. The second cycle was applied two week later (Day 21, 24, 27).

### Detection of suppressive cytokine IL-10 and TGF-β

The expression level of IL-10 and TGF-β were measured from rabbit PBMCs and spleens on day 2 after last immunization (day 28).

Specific procedures are: 1) The PBMCs from immunized rabbits were isolated and purified by Ficoll, in which 4ml of Ficoll400 (Sigma) were added under the 8ml of rabbit blood and spin down at 1500rpm for 15 minutes. The PBMC phase was taken out and washed after spin down. Then the PBMC were stimulated with 10 µg/ml of rh-insulin in vitro. 2) The spleen cells from immunized rabbit were collected and red blood cell removed by adding 2ml lysis buffer (Biyuntian) into cells suspension for 2 minutes. Adding equal volume of FBS to stop the lysis reaction. The spleen cells were counted and stimulated with 10µg/ml of rh-insulin. 3) 24 hours later of stimulation, the stimulated cells and PBMC were collected and Trizol added into the cell solution for RNA extraction. 4) The RNA were purified and transcribed into cDNA by Toyobo ReversTraAce kit according to the instruction. 5) The primers for HPRT, IL-10 and TGF-β amplification were designed as bellows:
HPRT p1: 5'-CCATCACATTGTAGCCCTCTGT-3'
HPRT p2: 5'-CTTGCGACCTTGACCATCTTT-3'
IL-10 p1: 5'-TATGTTGCCTGGTCTTCCTGG5-3'
IL-10 p2: 5'-CTCCACTGCCTTGCTCTTGT-3'
TGF-β p1: 5'-AACAAGAGCAGAAGGCGAATG-3'
TGF-β p2: 5'-ACAGCAAGGAGAAGCGGATG-3'

The endogenous gene encoding the HPRT as a reference gene was amplified and adjusted to the same level for each sample. 6) The IL-10 and TGF-β were amplified by PCR and the expressions were analyzed by DNA gel running and Gelpro software.

The result in Figure 12 shows that group 3 (10 + 1) injected with 10 µg of rh-insulin plus 1µg of DEX can induce highest and less high amount of IL-10 and TGF-β in spleen and PBMC in the treated animals.

### Example 5. Dose effect of drug compositions in dogs.

It demonstrated in example 4 that rh-insulin and DEX have therapeutic effect against TID in rabbit, then the effect was evaluated in dogs.

### Immunization of dogs.

Dogs used to induce TID model were injected with 60mg/kg Alloxan (Sigma) on day -3. The level of blood glucose was monitored. A successful dog TID model was defined as its blood glucose level reach to 12mM or higher in two consecutive days. The drug was injected i.p on days 1, 4 and 7 as one treatment cycle. The second cycle was applied after two weeks later. Nine dogs with 15-20 kg were divided into 3 groups with 3 per group. The group 1 (100 + 15) was treated with 100µg of rh-insulin plus 15 µg of DEX. Group 2 (100 + 1.5) was treated with 100µg of rh-insulin plus 1.5µg of DEX. Group 3 were diabetic group. The second cycle was applied two week later (Treated on day 21, 24,27).

### Detecting levels of IL-10, TGF-β and Treg in dogs.

### 1. Detection of IL-10, TGF-β in dog.

The IL-10 and TGF-β expression were detected in drug treated dogs on day-3, 0, 8, 20 and 28.

Specific procedures are: 1) The PBMCs from immunized dogs were isolated and purified by Ficoll, in which 4ml of Ficoll400 (Sigma) were added under the 8ml of dog blood and spin down at 1500rpm for 15 minutes. The PBMC phase was taken out and washed after spin down. Then the PBMC were stimulated with 10 µg/ml of rh-insulin in vitro. 2) 24 hours later of stimulation, the stimulated cells were collected and Trizol added into the cell solution for RNA extraction. 3) The RNA were purified and transcribed into cDNA by Toyobo ReversTraAce kit according to the instruction. 4) The primers for HPRT, IL-10 and TGF-β amplification were designed as bellows:
HPRT p1: 5'-AGCTTGCTGGTGAAAAGGAC-3'
HPRT p2: 5'-TTATAGTCAAGGGCATATCC-3'
IL-10 p1: 5'-ATGCATGGCTCAGCACCGCT-3'
IL-10 p2: 5'-TGTTCTCCAGCACGTTTCAGA-3'
TGF-β p1: 5'-TGGAACTGGTGAAGCGGAAG-3'
TGF-β p2: 5'-TTGCGGAAGTCAATGTAGAGC-3'
The endogenous gene encoding the HPRT as a reference gene was amplified and adjusted to the same level for each sample. 5) The IL-10 and TGF-β were amplified by PCR and the expressions were analyzed by DNA gel running and Gelpro software. The dogs without diabetic symptom were set up as control group.

The result in figure 13 shows that compositions treatment significantly up-regulate the level of IL-10 and TGF-β in dogPBMCs. However, less dose dependent effects were observed (fig 13).

### 2. Detection of Treg in dogs.

The Treg populations were detected on day 2 after immunization. The percentage of Treg can reflect that compositions treatment could induce Treg.

The pancreatic cells prepared 2 days after final immunization (day 28). The specific procedures are: 1) The dogs were sacrificed 100ml 1mg/ml of Collagenase P (Roche, Cat. No. 11213857001) were injected into pancreatic tissue and digested for 1 hour at 37°C. 2) The pancreatic cells were washed twice and spin down at 250g for 1 minute. 3) The cell pellets were re-suspended with 15ml of 25% Ficoll (Roche) and in turns 9ml of 23% Ficoll, 6ml of 20% Ficoll, 6ml of 11% Ficoll were added into above the 23% Ficoll. Then spin down the cell solution at 800g for 10 minutes and the islet cell phase were taken out, the Ficoll solution in islet cell was washed out with twice PBS washing. 4) The islets were digested with 0.25% of trypsin for for 10 minutes at 37°C. 5) The single suspension islet cells were stained with surface marker of CD4 (FITC-anti-CD4, eBioscience11-5040) and intracellular protein of Foxp3 (PE-anti-Foxp3, eBioscience 12-5773), then the stained markers were analyzed by FACs

The result in Figure 14 shows that the percentage of Treg to CD4 cells increased whereas the percentage of Treg to all cells significantly increased in group 2 (100 + 1.5).

### Example 6. Evaluation of treatment effect on TID dogs.

The compositions (rh-Insulin + Dex) and dosing were examined in the Example 5, the effect was further evaluated on compositions of rh-insulin plus CsA.

### Dog diabetic model induction and immunization.

Induction of dog type I diabetic model were according to the Example 5, 15 dogs with 15kg body weight were injected with 60mg/kg Alloxan (Sigma) on day -5. The level of blood glucose was monitored. A successful dog TID model was defined as its blood glucose level reach to 12mM or higher in two consecutive days. The dogs were divided into 3 groups at 5 per group and injected i.p on days 1, 4 and 7 as one treatment cycle. The group 1 were injected 100ug rh-insulin (0.15IU/kg body weight) and 15ug of Dex (1ug/kg body weight) in 100ul of PBS; group 2 were injected with 100ug rh-insulin and 1.5mg of CsA (100ug/kg body weight) in 100ul of PBS; group 3 were injected with 100ul of PBS only for the disease model. The second treatment cycle was applied one week later (day 21, 24, 27).

### Changes of survival, level of blood glucose and body weights in dogs.

### 1. Detection of Survival in dogs

Dog survives will reflect quality of dog life and efficacy of the treatments. Survived dogs were counted and calculated after the treatments.

As showed in Figure 15, all dogs dead in the group 3, 2 survived in the group 1 on day 21 (40% of survival), 3 survived in the group 2 on day 21 (60% survival). This evaluation demonstrated that the compositions and methods of rh-insulin plus Dex or rh-insulin plus CsA increased the chance survival for these TID dogs.

### 2. Detection of level change of blood glucose.

To examine changing level of blood glucose would reflect efficacy of the treatment.

Test procedure: 10 ul of blood samples were spotted on the test strips and the test instrument read the concentration of blood glucose.

The result showed that level of blood glucose were relatively regulated in the group 1 with some of fluctuation (Figure 16, indicated as the solid line); level of blood glucose were complete regulated in group 2 at range of 10-15mmol (Figure 17, indicated as the solid line), whereas, all animals dead in group 3 (Figures 16 and 17, indicated as the dashed lines).

### 3. Detection of body weight change in dogs

To examine changing body weights would reflect efficacy of the treatment.

As showed in Figure 18, reduction of body weight were control in some degree in group 1, completely control in group 2. Whereas group 3 all dead.

### Example 7. The drug effects on human DC conversion from TID patents' PBMC.

The drug effect was further evaluated on human DC conversion after human PBMCs treated with the drug compositions.

### PBMC collecting and processing from human blood.

Three blood samples from normal human individuals and 2 blood samples from TID patients were collected at 10 ml for each. The PBMCs were isolated by Ficoll, in which 4ml of Ficoll400 (Sigma) were added under the 8ml of blood and spin down at 1500rpm for 15 minutes. The PBMC phase was taken out and washed after spin down. Then the PBMC were stimulated with rhGM-CSF and rhlL-4 (R&D System) for 3 days. The induced DC were seeded into 96-well plate for 2×10⁶ cells per well and further stimulated with 1) 10µg/ml of rh-insulin, 2) 10µg/ml of DEX, 3) 10µg/ml of rh-insulin plus DEX.

### Detection of DC related markers and suppressive IL-10 level

### 1. Detection of CD40, CD80, CD83, CD86, MHC-II on DCs.

The surface markers, CD40, CD80, CD83, CD86, MHC-II on DC, were detected after 3 days stimulation.

The inhibition effect by drug treatment on DC maturation was determined as in following protocols. 1) The DCs were stained with different cell marker combos, and listed as following, CD1a-FITC (eBioscience, 11-0019), CD40-PE(eBioscience, 12-0409), CD1a-FITC(eBioscience, 11-0019) and CD80-PE(eBioscience, 12-0809), CD1a-FITC(eBioscience, 11-0019), D83-PE(Biolegend, 305322), CD1a-FITC(eBioscience, 11-0019), CD86-PE(eBioscience, 12-0869), CD1a-FITC (eBioscience, 11-0019) and MHC-II-PE. In which, the 0.25µl of each antibody per 10⁶ cells was premixed and added. 2) The stained cells were detected by FACs after 10 minutes staining and washings.
The result in Figure 19A shows that the CD40 expression was down-regulated on DC from TID patient's and normal human PBMCs after the drug treatment. CD40 down regulation would indicate that decreased matured DC could convert naïve T cells into Treg cells. 2. Detection of suppressive IL-10 level.

The IL-10 expression detected after 3 days stimulation.

The detail procedures are: 1) The 30ul supernatant of cells were collected and mixed with 30 µl PBS which contained 0.1µl FlexSet microbead for 30 minutes. The 30 µl PBS that contained 0.1µl anti-IL-10 PE antibody were added and incubated for another 30 minutes. 2) The IL-10 expressions were measured by FACs.

The result in Figure 19B shows that the IL-10 expressions were significantly up-regulated after the drug treatment.

### Example 8. The screening of immune-suppressive agents on human PBMC to DC conversions.

The results of Example 6 indicate that DC can be induced from human PBMCs after compositions treatment. The other immune suppressive agents were further screened to enhance the therapeutic effect of compositions.

The immune-suppressive agents in this example are DEX, Rap, CsA and FK506.

Experimental materials; 25 blood samples from TID patients were collected at 10 ml of each sample. The PBMCs were isolated by Ficoll, in which 4ml of Ficoll400 (Sigma) were added under the 8ml of blood and spin down at 1500rpm for 15 minutes. The PBMC phase was taken out and washed after spin down. Then the PBMC were stimulated with rhGM-CSF and rhlL-4 (R&D System) for 3 days. The induced DC were seeded into 96-well plate for 2×10⁶ cells per well and further stimulated with 1) 10µg/ml of rh-insulin, 2) 10µg/ml of rh-insulin plus DEX, 2) 10µg/ml of rh-insulin plus Rap, 4) 10µg/ml of rh-insulin plus CsA, 5) 10µg/ml of rh-insulin plus FK506.

### Detection of DC related markers and suppressive IL-10 level

### 1. Detection of CD40, CD80, CD83, CD86, MHC-II on DC.

The surface markers, CD40, CD80, CD83, CD86, MHC-II on DC were detected after 3 days stimulation.

The effects of different drug compositions on DC maturation were determined as following protocols.
1) The stimulated DC were stained with different cell marker combo, CD1a-FITC (eBioscience, 11-0019) and CD40-PE (eBioscience, 12-0409), CD1a-FITC (eBioscience, 11-0019) and CD80-PE (eBioscience, 12-0809), CD1a-FITC (eBioscience, 11-0019) and D83-PE (Biolegend, 305322), CD1a-FITC (eBioscience, 11-0019) and CD86-PE (eBioscience, 12-0869), CD1a-FITC (eBioscience, 11-0019) and MHC-II-PE; In which, the 0.25µl of each antibody per 10⁶ cells were added. 2) The stained cells were detected by FACs after 10 minutes staining and washing.

The result shows that the expressions of CD40, CD80, CD80 and CD86 were down regulated after the drug treatment. The most effective drugs are the DEX and CsA in comparisons. (Fig 20)

### Industry application

_Vaccine compositions and their use for treatment or/and prevention of TID is disclosed in this invention. They can increase the ratio of CD4⁺CD25⁺Treg to CD4⁺T cells; enhance the proliferation of CD4⁺CD25⁺ Treg; increase the IL-10 secretion in T cells; control the blood glucose; inhibit the cytotoxicity effect of auto-reactive CD8⁺T cells; up regulate the transcription level of IL-10 or /and TGF-β in PBMC or/ and spleen cells; inhibit the DC maturation; to induce immune suppression. As the consequence, the TID can be effectively cured.
## Claims

1. A composition suitable for treatment and/or prevention of type 1 diabetes (T1D), comprising as the active ingredient of this composition 1) or 2):
1) Mixture of protein antigen and immune suppressive agent(s), wherein the weight ratio of the protein antigen and the immune suppressive agent(s) is 1:1 to 10:1,
2) Mixture of epitope peptides of said protein antigen and immune suppressive agent(s), wherein the weight ratio of the epitope peptides and the immune suppressive agent(s) is 1:1,
wherein the protein antigen is Insulin or a fragment thereof, and
wherein the immune suppressive agent is selected from the group consisting of dexamethasone (Dex), tacrolimus (FK506), cyclosporine (CsA), prednisone (Pred), methylprednisolone (MP), and combinations thereof.

2. The composition of claim 1, wherein the immunosuppressive agent is dexamethasone (Dex).

3. The composition of claim 1, wherein the sequence of insulin and fragments thereof are derived from human, dog or cat and the epitope peptides are derived from human, cat and dog native protein or are chemically synthesized in vitro.

4. The composition of claim 3, wherein the listed epitopic sequence from insulin refers to sequence 1, or sequence 2, or sequence 3, or sequence 12, or sequence 13; the listed epitope peptide from dog insulin refers to sequence 4, or sequence 5, or sequence 12, or sequence 13; the listed epitope peptide from cat insulin refers to sequence 6, or sequence 7, or sequence 12, or sequence 13.

5. The composition of claims 1-4 for use in a method of type 1 diabetes treatment and/or prevention.

6. The composition of claims 1-5 for use in a method of treatment and/or prevention of T1D meeting at least one application listed in below:
(1) Increasing the ratio of CD4+ CD25+Treg to CD4+T cells;
(2) Enhancing the proliferation of CD4+ CD25+ Treg cells;
(3) Increasing the IL-10 secretion from T cells;
(4) Controlling the blood glucose for T1 D patients;
(5) Inhibiting the cytotoxicity effect of auto-reactive CD8⁺ T cells;
(6) Up-regulating transcription level of IL-10 and/or TGF-β in PBMC and/or spleen;
(7) Inhibiting the DC maturation;
(8) Decreasing the expression of at least one protein of CD40, CD80, CD83, CD86 in DC.

## Patentansprüche

1. Eine Zusammensetzung, die zur Behandlung und/oder Prophylaxe von Typ I Diabetes (T1D) geeignet ist, wobei die Zusammensetzung als aktiven Bestandteil dieser Zusammensetzung 1) oder 2):
1) Mischung eines Proteinantigens und von immunsuppressivem/n Agens/Agenzien, wobei das Gewichtsverhältnis des Proteinantigens und des/der immunsuppressivem Agens/Agenzien 1:1 bis 10:1 ist,
2) Mischung von Epitop-Peptiden dieses Proteinantigens und von immunsuppressivem/n Agens/Agenzien, wobei das Gewichtsverhältnis der Epitop-Peptide und von immunsuppressivem/n Agens/Agenzien 1:1 ist,
umfasst, wobei das Proteinantigen Insulin oder ein Fragment davon ist, und
wobei das immunsuppressive Agens aus der Gruppe, bestehend aus Dexamethason (Dex), Tacrolimus (FK506), Cyclosporin (CsA), Prednison (Pred), Methylprednisolon (MP) und Kombinationen davon, ausgewählt ist.

2. Die Zusammensetzung nach Anspruch 1, wobei das immunsuppressive Agens Dexamethason (Dex) ist.

3. Zusammensetzung nach Anspruch 1, wobei die Sequenz von Insulin und Fragmenten davon abgeleitet ist aus Mensch, Hund oder Katze und die Epitop-Peptide abgeleitet sind aus nativem menschlichen, Katzen- und Hunde-Protein oder chemisch in vitro synthetisiert sind.

4. Die Zusammensetzung nach Anspruch 3, wobei die gelistete Epitop-Sequenz von Insulin sich auf Sequenz 1 oder Sequenz 2 oder Sequenz 3 oder Sequenz 12 oder Sequenz 13 bezieht; wobei das gelistete Epitop-Peptid von Hunde-Insulin sich auf Sequenz 4, oder Sequenz 5, oder Sequenz 12 oder Sequenz 13 bezieht; wobei das gelistete Epitop-Peptid von Katzen-Insulin sich auf Sequenz 6 oder Sequenz 7 oder Sequenz 12 oder Sequenz 13 bezieht.

5. Zusammensetzung nach den Ansprüchen 1 bis 4 zur Verwendung bei einem Verfahren zur Behandlung und/oder Prophylaxe von Typ 1 Diabetes.

6. Zusammensetzung nach den Ansprüchen 1 bis 5 zur Verwendung in einem Verfahren zur Behandlung und/oder Prophylaxe von T1D, wobei das Verfahren mindestens eine der unten genannten Anwendungen betrifft:
(1) Erhöhung des Verhältnisses von CD4+ CD25+Treg gegenüber CD4+T-Zellen;
(2) Verstärkung der Proliferation von CD4+ CD25+ Treg-Zellen;
(3) Erhöhung der IL-10 Sekretion von T-Zellen;
(4) Kontrolle der Blutglukose bei T1D-Patienten;
(5) Inhibition des zytotoxischen Effekts von auto-reaktiven C08⁺ T-Zellen;
(6) Hochregulation des Transkriptionsniveaus von IL-10 und/oder TGF-β in PBMC und/oder Milz;
(7) Inhibition der DC-Maturierung;
(8) Abnahme der Expression von mindestens einem Protein von CD40, CD80, CD83, CD86 in DC.

## Revendications

1. Composition adaptée au traitement et/ou à la prévention du diabète de type 1 (T1D), comprenant 1) ou 2) comme principe actif de cette composition :
1) un mélange d'antigène protéique et d'agent(s) immunosuppresseur(s), dans lequel le rapport pondéral de l'antigène protéique et du ou des agents immunosuppresseurs est de 1:1 à 10:1,
2) un mélange de peptides épitopes dudit antigène protéique et d'agent(s) immunosuppresseur(s), dans lequel le rapport pondéral des peptides épitopes et du ou des agents immunosuppresseurs est de 1:1,
dans lequel l'antigène protéique est l'insuline ou un fragment de celle-ci, et dans lequel l'agent immunosuppresseur est choisi dans le groupe constitué par la dexaméthasone (Dex), le tacrolimus (FK506), la cyclosporine (CsA), la prednisone (Pred), la méthylprednisolone (MP) et leurs combinaisons.

2. Composition selon la revendication 1, dans laquelle l'agent immunosuppresseur est la dexaméthasone (Dex).

3. Composition selon la revendication 1, dans laquelle la séquence d'insuline et de fragments de celle-ci est dérivée de l'homme, du chien ou du chat, et les peptides épitopes sont dérivés d'une protéine native humaine, de chat et de chien ou sont synthétisés par voie chimique in vitro.

4. Composition selon la revendication 3, dans laquelle la séquence épitopique répertoriée provenant d'insuline fait référence à la séquence 1, ou à la séquence 2, ou à la séquence 3, ou à la séquence 12, ou à la séquence 13 ; le peptide épitope répertorié provenant de l'insuline de chien fait référence à la séquence 4, ou à la séquence 5, ou à la séquence 12, ou à la séquence 13 ; le peptide épitope répertorié provenant de l'insuline de chat fait référence à la séquence 6, ou à la séquence 7, ou à la séquence 12, ou à la séquence 13.

5. Composition selon les revendications 1 à 4, pour une utilisation dans une méthode de traitement et/ou de prévention du diabète de type 1.

6. Composition selon les revendications 1 à 5, pour une utilisation dans une méthode de traitement et/ou de prévention du T1D répondant à au moins une application énumérée ci-dessous :
(1) l'augmentation du rapport des cellules Treg CD4+ CD25+ aux lymphocytes T CD4+ ;
(2) le renforcement de la prolifération des cellules Treg CD4+ CD25+ ;
(3) l'augmentation de la sécrétion de l'IL-10 par les lymphocytes T ;
(4) la régulation de la glycémie chez les patients atteints de T1D ;
(5) l'inhibition de l'effet cytotoxique des lymphocytes T CD8⁺ auto-réactifs ;
(6) la régulation à la hausse du niveau de transcription de l'IL-10 et/ou du TGF-β dans les PBMC et/ou la rate ;
(7) l'inhibition de la maturation des DC ;
(8) la diminution de l'expression d'au moins une protéine de CD40, CD80, CD83, CD86 dans les DC.


<110> BEIJING ADVACCINE BIOTECHNOLOGY CO., LTD.
<120> Vaccine compositions and methods for treatment and /or prevention of Type I diabetes
<130> PCGNAK13009
<160> 13
<170> PatentIn version 3.5
<210> 1
   <211> 15
   <212> PRT
   <213> artificial
<220>
   <223>
<400> 1
   Ser His Leu Val Glu Ala Leu Tyr Leu Val Cys Gly Glu Arg Gly
   1               5                   10                  15
<210> 2
   <211> 9
   <212> PRT
   <213> artificial
<220>
   <223>
<400> 2
   Leu Tyr Leu Val Cys Gly Glu Arg Gly
   1               5
<210> 3
   <211> 31
   <212> PRT
   <213> artificial
<220>
   <223>
<400> 3
   Glu Ala Glu Asp Leu Gln Val Gly Gln Val Glu Leu Gly Gly Gly Pro
   1               5                   10                  15
   Gly Ala Ser Gly Leu Gln Pro Leu Ala Leu Glu Gly Ser Leu Gln
               20                  25                  30
<210> 4
   <211> 15
   <212> PRT
   <213> artificial
<220>
   <223>
<400> 4
   Ser His Leu Val Glu Ala Leu Tyr Leu Val Cys Gly Glu Arg Gly
   1               5                   10                  15
<210> 5
   <211> 9
   <212> PRT
   <213> artificial
<220>
   <223>
<400> 5
   Leu Tyr Leu Val Cys Gly Glu Arg Gly
   1               5
<210> 6
   <211> 15
   <212> PRT
   <213> artificial
<220>
   <223>
<400> 6
   Ser His Leu Val Glu Ala Leu Tyr Leu Val Cys Gly Glu Arg Gly
   1               5                   10                  15
<210> 7
   <211> 9
   <212> PRT
   <213> artificial
<220>
   <223>
<400> 7
   Leu Tyr Leu Val Cys Gly Glu Arg Gly
   1 5
<210> 8
   <211> 10
   <212> PRT
   <213> artificial
<220>
   <223>
<400> 8
   Val Met Asn Ile Leu Leu Gln Tyr Val Val
   1 5 10
<210> 9
   <211> 37
   <212> PRT
   <213> artificial
<220>
   <223>
<400> 9
<210> 10
   <211> 37
   <212> PRT
   <213> artificial
<220>
   <223>
<400> 10
   Lys Cys Asn Thr Ala Thr Cys Ala Thr Gln Arg Leu Ala Asn Phe Leu
   1 5 10 15
   Val Arg Thr Ser Asn Asn Leu Gly Ala Ile Leu Ser Pro Thr Asn Val
   20 25 30
   Gly Ser Asn Thr Tyr
   35
<210> 11
   <211> 37
   <212> PRT
   <213> artificial
<220>
   <223>
<400> 11
   Lys Cys Asn Thr Ala Thr Cys Ala Thr Gln Arg Leu Ala Asn Phe Leu
   1 5 10 15
   Ile Arg Ser Ser Asn Asn Leu Gly Ala Ile Leu Ser Pro Thr Asn Val
               20                  25                  30
   Gly Ser Asn Thr Tyr
           35
<210> 12
   <211> 17
   <212> PRT
   <213> artificial
<220>
   <223>
<400> 12
   Gly Phe Phe Tyr Thr Pro Lys Thr Arg Arg Glu Ala Glu Asp Leu Gln
   1               5                   10                  15
   Val
<210> 13
   <211> 27
   <212> PRT
   <213> artificial
<220>
   <223>
<400> 13
   His Leu Val Glu Ala Leu Tyr Leu Val Cys Gly Glu Arg Gly Phe Phe
   1               5                   10                  15
   Tyr Thr Pro Lys Thr Arg Arg Glu Ala Glu Asp
               20                  25